# EUROPEAN PATENT SPECIFICATION

(11) **EP 3 850 070 B1**
(45) Date of publication and mention of the grant of the patent: **06.11.2024**
(21) Application number: 19762433.1
(22) Date of filing: 06.09.2019
(51) Int. Cl.: C11D 3/28, A47L 13/17, A01N 37/44, C11D 17/04, C07D 207/44, C07D 207/38, A01N 43/36, A01N 57/20

(54) **WIPE**
WISCHTUCH
LINGETTE

(30) Priority: 14.09.2018 EP 18194538
(43) Date of publication of application: 21.07.2021
(73) Proprietor: UNILEVER GLOBAL IP LIMITED, Wirral Merseyside CH62 4ZD (GB); Unilever IP Holdings B.V., 3013 AL Rotterdam (NL)
(72) Inventor: KOTSAKIS, Panagiotis, 145 76 Athens (GR); PARRY, Neil, James, Wirral Merseyside CH63 3JW (GB); MOORE, Simon, John, Wirral Merseyside CH63 3JW (GB)
(74) Representative: McHugh, Paul Edward
(86) International application number: PCT/EP2019/073902
(87) International publication number: WO 2020/053106

(56) References cited:
- WO-A1-2007/085042
- WO-A1-2017/029070
- WO-A2-02/40628

## Description

### Field of Invention

The invention relates to a wipe suitable for application to a hard surface.

### Background of the Invention

Wipes are commonly used as surface care products. They can be commonly used to clean a surface using for example a detergent, or to wipe a surface using for example an antimicrobrial ingredient.

WO 02/40628 discloses wipes, articles of manufacture and methods for cleaning and/or sanitizing/disinfecting, foods, food contact surfaces, toys and/or infant contact surfaces, especially for the purpose of making food safe for human consumption, and/or toys and/or infant contact surfaces making safe for children, safe for use by and around children.

WO 2007/085042 discloses lactams, methods for their synthesis and uses of these compounds.

WO 2017/029070 discloses encapsulated lactams. The encapsulated lactams are suitable for use in compositions, for example, in antimicrobial, anti-biofilm and bacteriostatic compositions.

There is a need for wipes that provide improved surface care benefits to treated surfaces.

### Summary of the Invention

We have found that by adding a lactam as described herein to the wipe, the resulting wipe provides surface care benefits to a wiped surface, for example the treated surface displays a reduced level of microbrial concentration.

Furthermore, we have found that the wipes are self-preserving. There is a reduced level of bacterial growth on the lactam wipes themselves when compared to non-lactam wipes. This characteristic can thus reduce or remove the necessity for inclusion of a separate preservative.

The invention relates in a first aspect to a wipe suitable for application to a hard surface comprising from 0.0001 to 5 wt.% of a lactam, wherein the lactam is a lactam selected from: 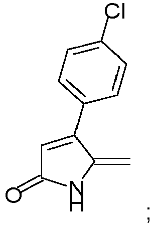 4-(4-chlorophenyl)-5-methylene-pyrrol-2-one; and 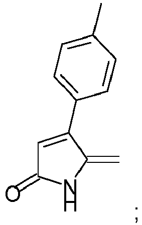 5-methylene-4-(p-tolyl)pyrrol-2-one.

Preferably the wipe comprises:-
a) from 0.0001 to 5 wt.% of a lactam; and,
b) from 0.1 to 20 wt.% of a surfactant.

More preferably the wipe comprises:-
a) from 0.0001 to 5 wt.% of a lactam;
b) from 0.1 to 20 wt.%, preferably from 0.5 to 10 wt.%, more preferably from 1 to 8 wt.% of a surfactant; and,
c) from 0.01 to 2.5 wt.% of a chelating agent.

It is preferred that the lactam is present at a level of from 0.0001 to 2.5 wt.%, more preferably from 0.0001 to 1 wt.%, most preferably from 0.001 to 1 wt.%.

More preferably the lactam is a lactam selected from: 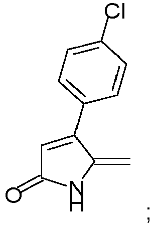 4-(4-chlorophenyl)-5-methylene-pyrrol-2-one; and

Preferably the lactam is in encapsulated form.

Preferably the surfactant is selected from anionic and nonionic surfactants, preferably nonionic surfactants, more preferably alcohol ethoxylates.

Preferably the chelating agent is citric acid; EDTA (ethylenediaminetetraacetic acid) or a phosphonic acid.

Preferably the wipe additionally comprises any one or more of the following: buffers, antifoam agents, perfumes, and, preservative.

In a second aspect, the invention relates to the use of a lactam in a wipe product to enhance preservation of said wipe product, wherein the lactam is selected from: 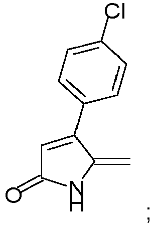 4-(4-chlorophenyl)-5-methylene-pyrrol-2-one; and 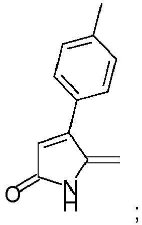 5-methylene-4-(p-tolyl)pyrrol-2-one.

In a third aspect, the invention relates to the use of a wipe according to the first aspect of the invention on a surface to reduce microorganism buildup on said surface.

Preferably in these uses of the lactam has the following structure: 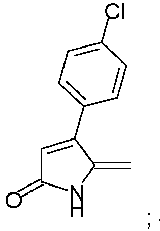 4-(4-chlorophenyl)-5-methylene-pyrrol-2-one.

### Detailed Description of the Invention

The indefinite article "a" or "an" and its corresponding definite article "the" as used herein means at least one, or one or more, unless specified otherwise.

It will be appreciated that, except where expressly provided otherwise, all preferences are combinable.

### Lactam

A lactam is a cyclic amide.

The lactam is a lactam selected from: 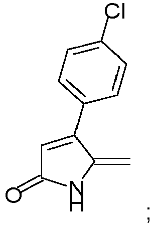 4-(4-chlorophenyl)-5-methylene-pyrrol-2-one; and 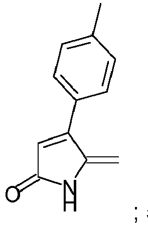 5-methylene-4-(p-tolyl)pyrrol-2-one.

The most preferred lactam is:- 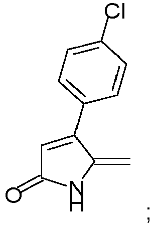 4-(4-chlorophenyl)-5-methylene-pyrrol-2-one.

Preferably the lactam is encapsulated.

Suitably, the encapsulated lactam is a polymer encapsulated lactam.

The encapsulated lactam may be encapsulated in a polymer selected from a poly urea polymer, a melamine-formaldehyde copolymer; a urea formaldehyde copolymer and mixtures thereof.

Suitably the polymer is a condensation polymer. For example, the polymer may be a condensation polymer of produced from a diamine and a disocyanate.

For example, the polymer may be or may comprise a polyurea of Formula P1: 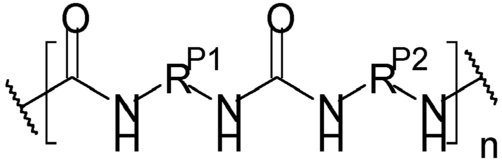 wherein R^{P1} comprises a phenylene and R^{P2} is an alkylene.

For example, R^{P1} may be -CH₂-phenylene; in other words, the polymer may be derived from polymethylene polyphenyl isocyanate.

For example, R^{P2} may be a straight chain alkylene of formula -(CH₂)ₘ-. In some cases, m is an integer from 2 to 10, for example from 2 to 8, for example from 4 to 8, for example, 6 (in other words, R^{P2} may be hexylene).

In other words, the lactam may be encapsulated in a polymer formed from polymethylene polyphenyl isocyanate and hexamethylenediamine.

In some cases, the polymer and / or encapsulate structure is selected and / or configured to permit controlled or triggered release. For example, the encapsulate may dissolve at a predetermined rate under certain conditions. For example, the encapsulate may release in response to a trigger. The trigger may be, for example, the presence or a certain concentration of acid, base, a salt, an enzyme; or a non-chemical trigger such as ultrasound or light.

Suitably, the lactam is encapsulated to form particles whose average diameter is from about 10 nanometers to about 1000 microns, preferably from about 50 nanometers to about 100 microns, more preferably from about 2 to about 40 microns, even more preferably from about 4 to 15 microns. A particularly preferred range is from about 5 to 10 microns, for example 6 to 7 microns. The capsule distribution can be narrow, broad or multimodal. Multimodal distributions may be composed of different types of capsule chemistries.

The encapsulation process suitably is done in a carrier oil, which may be a ketone. For example, the carrier oil may be a C₅₋₂₀alkyl ketone, for example a C₅₋₁₅alkyl ketone, for example a C₅₋₁₀alkyl ketone, for example a C₆₋₈alkyl ketone, such as a C₇alkyl ketone. The alkylketone may be branched or straight-chain. Preferably, it is straight chain. The oxo group of the alkyl ketone may be located at C2; in other words, the alkylketone may be an alkyl-2-one. A preferred carrier oil is 2-heptanone.

### Levels of lactam

Preferably the lactam is present at a level of from 0.0001 to 2.5 wt.%, preferably from 0.0001 to 1 wt.%. For example, the lactam may be suitably present at levels of 0.001 to 1 wt.%, or even 0.01 to 1 wt.%, or even 0.01 to 0.5 wt.%.

### Surfactant

The wipe preferably comprises from 0.1 to 20 wt.%, more preferably from 0.5 to 10 wt.%, most preferably from 1 to 10 wt.%, or even from 1 to 8 wt.%, for example from 2 to 8 wt.% of a surfactant.

Any suitable surfactant may be used, including for example, anionic, nonionic, cationic and amphoteric surfactants.

Preferably the surfactant is selected from anionic and nonionic surfactants.

Suitable anionic detergent compounds which may be used are usually water-soluble alkali metal salts of organic sulphates and sulphonates having alkyl radicals containing from about 8 to about 22 carbon atoms, the term alkyl being used to include the alkyl portion of higher alkyl radicals.

Examples of suitable synthetic anionic detergent compounds are sodium and potassium alkyl sulphates, especially those obtained by sulphating higher C₈ to C₁₈ alcohols, produced for example from tallow or coconut oil, sodium and potassium alkyl C₉ to C₂₀ benzene sulphonates, particularly sodium linear secondary alkyl C₁₀ to C₁₅ benzene sulphonates; and sodium alkyl glyceryl ether sulphates, especially those ethers of the higher alcohols derived from tallow or coconut oil and synthetic alcohols derived from petroleum.

The anionic surfactant is preferably selected from: linear alkyl benzene sulphonate; alkyl sulphates; alkyl ether sulphates; soaps; alkyl (preferably methyl) ester sulphonates, and mixtures thereof.

The most preferred anionic surfactants are selected from: linear alkyl benzene sulphonate; alkyl sulphates; alkyl ether sulphates and mixtures thereof. Preferably the alkyl ether sulphate is a C₁₂-C₁₄ n-alkyl ether sulphate with an average of 1 to 3EO (ethoxylate) units.

Sodium lauryl ether sulphate is particularly preferred (SLES). Preferably the linear alkyl benzene sulphonate is a sodium C₁₁ to C₁₅ alkyl benzene sulphonates. Preferably the alkyl sulphate is a linear or branched sodium C₁₂ to C₁₈ alkyl sulphates. Sodium dodecyl sulphate is particularly preferred, (SDS, also known as primary alkyl sulphate).

It is possible for two or more anionic surfactants to be present, for example linear alkyl benzene sulphonate together with an alkyl ether sulphate.

Suitable nonionic detergent compounds which may be used include, in particular, the reaction products of compounds having an aliphatic hydrophobic group and a reactive hydrogen atom, for example, aliphatic alcohols, acids or amides, especially ethylene oxide either alone or with propylene oxide. Preferred nonionic detergent compounds are the condensation products of aliphatic C₈ to C₁₈ primary or secondary linear or branched alcohols with ethylene oxide.

Preferably the surfactants used are saturated.

More preferably the surfactant comprises nonionic surfactants, more preferably alcohol ethoxylates.

Most preferably the nonionic surfactant is a C₈ to C₁₈ primary alcohol with an average ethoxylation of 7EO to 9EO units.

### Water

The wipe preferably includes water. Preferred levels of water range from 50 to 98 wt.%, morepreferably 75 to 95 wt.%

### Chelant

A chelating agent is preferably included in the wipe. Typical inclusion levels are from 0.01 to 2.5 wt.%, preferably 0.025 to 1 wt.%.

The chelant may preferably be: citric acid; EDTA (ethylenediaminetetraacetic acid) or a phosphonic acid.

Example phosphonic acid (or phosphonate salt thereof) chelating agents are: 1-Hydroxyethylidene-1,1-diphosphonic acid (HEDP); Diethylenetriaminepenta(methylenephosphonic acid) (DTPMP); Hexamethylenediaminetetra(methylenephosphonic acid) (HDTMP); Aminotris(methylenephosphonic acid) (ATMP); Ethylenediaminetetra(methylenephosphonic acid) (EDTMP); Tetramethylenediaminetetra(methylenephosphonic acid) (TDTMP); and, Phosphonobutanetricarboxylic acid (PBTC); and salts thereof.

Preferably the wipe additionally comprises any one or more of the following: buffers, antifoam agents, perfumes, and, preservative.

The invention will be further described with the following non-limiting examples.

### Examples

### Example 1 - Preparation of examples of preferred lactams

### Preparation of 4-(4-chlorophenyl)-5-hydroxy-5-methylfuran-2(5H)-one

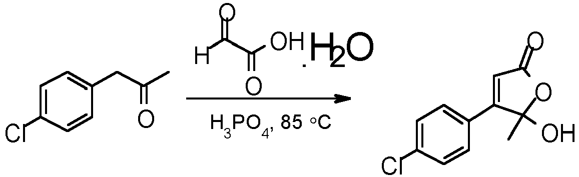

1-(4-Chlorophenyl)propan-2-one (40.00 g, 34.75 mL, 237.2 mmol), glyoxylic acid monohydrate (32.75 g, 355.8 mmol) and phosphoric acid (69.74 g, 711.7 mmol) were combined at room temperature before heating to 85 °C overnight. After cooling to room temperature, the mixture was poured into a mixture of water (500 mL) and ethyl acetate (500 mL). The layers were separated and the aqueous phase extracted with ethyl acetate (500 mL). The combined organic layers were washed with a 1:1 mixture of water and brine (2 x 500 mL), dried (MgSO₄) and concentrated under reduced pressure to yield 4-(4-chlorophenyl)-5-hydroxy-5-methylfuran-2(5H)-one (66.00 g, >100% yield) as a brown oil. The material was used in the next step without further purification.

### Preparation of 4-(4-chlorophenyl)-5-hydroxy-5-methyl-1H-pyrrol-2(5H)-one

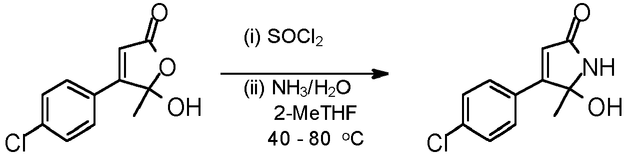

4-(4-Chlorophenyl)-5-hydroxy-5-methylfuran-2(5H)-one (66.00 g, 293.8 mmol) was dissolved in thionyl chloride (196.8 g, 120.0 mL, 1654 mmol) and heated at 40 °C for 1 hour, then 80 °C for 2 hours. The mixture was concentrated under reduced pressure and azeotroped with 2-methyltetrahydrofuran (200 mL). The residue was diluted with 2-methyltetrahydrofuran (160 mL) and this solution added to a cooled stirring mixture of 28% ammonia in water (180 mL) in 2-methyltetrahydrofuran (20 mL) at 0 °C. The mixture was warmed to room temperature and stirred overnight. Water (100 mL) and ethyl acetate (200 mL) were added and the layers separated. The aqueous phase was extracted with ethyl acetate (200 mL), and the combined organic extracts dried (MgSO₄) and concentrated under reduced pressure. Purification by dry flash column chromatography (5-60% ethyl acetate in heptane) yielded 4-(4-chlorophenyl)-5-hydroxy-5-methyl-1H-pyrrol-2(5H)-one (23.18 g, 35% yield) as a cream coloured solid.
¹H NMR (400 MHz, d₆-DMSO) 8.55 (brs, 1H), 7.88-7.83 (m, 2H), 7.51-7.46 (m, 2H), 6.37 (d, 1H), 6.32 (s, 1H), 1.45 (s, 3H)
UPLC (Basic) 1.51/5.00 min, 100% purity, M+H⁺ 224
MP 177 °C

### Preparation of 4-(4-chlorophenyl)-5-methylene-1H-pyrrol-2(5H)-one

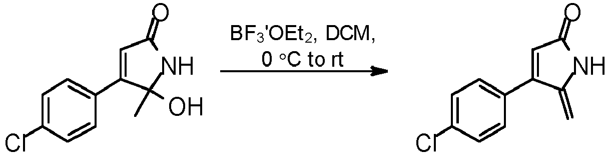

To a cooled solution of 4-(4-chlorophenyl)-5-hydroxy-5-methyl-1H-pyrrol-2(5H)-one (10.00 g, 44.51 mmol) in dry dichloromethane (100 mL) at 0 °C was added a solution of boron trifluoride diethyl etherate (8.213 g, 7.142 mL, 57.87 mmol) in dry dichloromethane (45 mL) over 15 minutes. The mixture was stirred at 0 °C, before slowly warming to room temperature and stirring for 2 hours. The reaction was quenched with ice-water (100 mL) and the layers separated. The aqueous layer was extracted with dichloromethane (100 mL), and the combined organic layers washed with a 1:1 mixture of water and saturated aqueous sodium hydrogen carbonate solution (100 mL), dried (MgSO₄) and filtered. Silica was added to the filtrate and the mixture stirred for 10 minutes before filtering through a plug of silica, washing through with dichloromethane followed by a 3:1 mixture of dichloromethane:diethyl ether. Fractions containing the desired product were combined and concentrated under reduced pressure. Upon concentration a precipitate formed, which was collected by filtration, washing with diethyl ether, to yield 4-(4-chlorophenyl)-5-methylene-1H-pyrrol-2(5H)-one (5.25 g, 57% yield) as a cream coloured solid.
¹H NMR (400 MHz, d₆-DMSO) 10.10 (s, 1H), 7.54-7.47 (m, 4H), 6.36 (s, 1H), 5.04 (t, 1H), 4.85 (s, 1H)
UPLC (Basic) 1.87/5.00 min, 100% purity, M+H⁺ 206
MP 182 °C

### Preparation of 5-hydroxy-5-methyl-4-(p-tolyl)furan-2(5H)-one

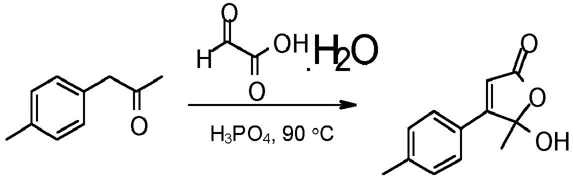

1-(p-Tolyl)propan-2-one (25.00 g, 24.00 mL, 168.7 mmol), glyoxylic acid monohydrate (23.29 g, 253.0 mmol) and phosphoric acid (49.60 g, 506.1 mmol) were combined at room temperature before heating at 90 °C overnight. After cooling to room temperature, the mixture was poured into a stirring mixture of ice-water (400 mL) and ethyl acetate (400 mL). The layers were separated and the organic phase washed with water (100 mL), dried (MgSO₄) and concentrated under reduced pressure. The mixture was azeotroped with 2-methyltetrahydrofuran (50 mL) to yield 5-hydroxy-5-methyl-4-(p-tolyl)furan-2(5H)-one (16.50 g, 48% yield) as a brown solid.
¹H NMR (400 MHz, d₆-DMSO) 7.86 (s, 1H), 7.75 (d, 2H), 7.28 (d, 2H), 6.59 (s, 1H), 2.32 (s, 3H), 1.61 (s, 3H)

### Preparation of 5-hydroxy-5-methyl-4-(p-tolyl)-1H-pyrrol-2(5H)-one

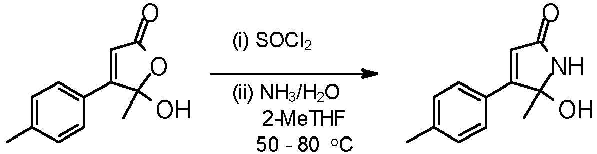

5-Hydroxy-5-methyl-4-(p-tolyl)furan-2(5H)-one (16.50 g, 80.80 mmol) was dissolved in thionyl chloride (48.06 g, 29.47 mL, 404.0 mmol) and heated at 50 °C for 1 hour, before heating at reflux for 1 hour. After cooling to room temperature, the mixture was concentrated under reduced pressure and azeotroped with 2-methyltetra-hydrofuran (2 x 50 mL). The residue was diluted with 2-methyltetrahydrofuran (60 mL) and this solution added to a cooled stirring mixture of 28% ammonia in water (55 mL, 808.0 mol) in 2-methyltetrahydrofuran (10 mL) at 0 °C. The mixture was warmed to room temperature and stirred overnight. 2-Methyltetrahydrofuran was removed under reduced pressure, and the residue diluted with water (200 mL) and diethyl ether (100 mL) and the mixture stirred for 20 minutes at room temperature. The solids were collected by filtration and stirred in water (100 mL) and diethyl ether (50 mL) at room temperature for 10 minutes. The solids were collected by filtration and washed with water, diethyl ether and dried under vacuum at 50 °C to yield 5-hydroxy-5-methyl-4-(p-tolyl)-1H-pyrrol-2(5H)-one (10.49 g, 31% yield) as a light beige solid.
¹H NMR (400 MHz, d₆-DMSO) 8.44 (brs, 1H), 7.73 (d, 2H), 7.21 (d, 2H), 6.24 (s, 2H), 2.29 (s, 3H), 1.45 (s, 3H)
¹³C NMR (400 MHz, d₆-DMSO) 170.4 (s, 1C), 161.1 (s, 1C), 139.8 (s, 1C), 129.7 (s, 2C), 128.9 (s, 1C), 128.2 (s, 2C), 119.1 (s, 1C), 87.8 (s, 1C), 26.7 (s, 1C), 21.5 (s, 1C)
UPLC (Basic) 1.41/5.00 min, 100% purity, M+H⁺ 204
MP 178 °C Decomposition

### Preparation of 5-methylene-4-(p-tolyl)-1H-pyrrol-2(5H)-one

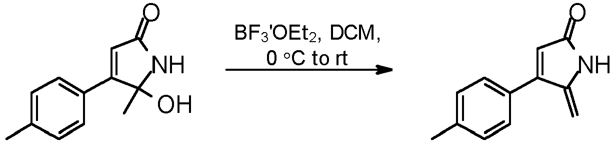

To a cooled solution of 5-hydroxy-5-methyl-4-(p-tolyl)-1H-pyrrol-2(5H)-one (8.68 g, 42.7 mmol) in dry dichloromethane (87 mL) at 0 °C was added a solution of boron trifluoride diethyl etherate (6.85 g, 5.96 mL, 55.5 mmol) in dry dichloromethane (40 mL) over 15 minutes. After 1 hour the mixture was allowed to slowly warm to room temperature. After a further 3 hours, the reaction was diluted with dichloromethane (50 mL) and ice-water (100 mL) and stirred for 10 minutes. The layers were separated and the organic layer washed with water (100 mL), a 1:1 mixture of water and saturated aqueous sodium hydrogen carbonate solution (100 mL) and brine (100 mL) and the organic layer filtered through Celite, washing with dichloromethane. Any excess water was removed by pipette before drying the filtrate (MgSO₄) and concentrating under reduced pressure to a brown solid. The solids were stirred in hot dichloromethane (120 mL) for 15 minutes before slowly cooling to room temperature and then 0 °C. The solids were collected by filtration to yield 5-methylene-4-(p-tolyl)-1H-pyrrol-2(5H)-one (3.87 g, 49% yield) as a yellow solid. Silica was added to the filtrate and the mixture stirred for 10 minutes before filtering through a plug of silica, washing through with dichloromethane and then a 4:1 mixture of dichloromethane:diethyl ether. The filtrate was concentrated under reduced pressure to yield 5-methylene-4-(p-tolyl)-1H-pyrrol-2(5H)-one (0.58 g, 7%) as a yellow solid. Total yield of 5-methylene-4-(p-tolyl)-1H-pyrrol-2(5H)-one (4.45 g, 56% yield).
¹H NMR (400 MHz, d₆-DMSO) 10.11 (brs, 1H), 7.35 (d, 2H), 7.25 (d, 2H), 6.25 (s, 1H), 5.01 (s, 1H), 4.85 (s, 1H), 2.31 (s, 3H)
UPLC (Basic) 1.83/5.00 min, 100% purity, M+H⁺ 186
MP 200 °C Decomposition

### Example 2 - Methods of manufacture of the lactam wipe

Lactam (4-(4-chlorophenyl)-5-methylene-pyrrol-2-one) is pre-mixed to a final concentration of 1mg/ml with neat solvent (ethanol, DMSO, or methoxymethyl butanol) until fully dispersed. The resulting lactam solution is the added to the base wipe formulation to a final lactam concentration of 100mg/l. Continuous wipe sheets are soaked in the formulation and excess is pressed away prior to cutting to smaller pieces and packaged.

The following wipe formulation was made:-

| **Ingredient** | **% wt.%** |
|---|---|
| Water | to 100 |
| Neodol 91-5 | 6.0 |
| Chelating Agent | 0.05 |
| Buffers | Qs |
| Antifoam agent | 0.08 |
| Lactam | 0.005 |
| Methoxy Methyl Butanol (MMB) | 1 |

### Example 3 - Examples of surface wipe analysis using the wipes

### Results of lactam wipes on E. coli

The wipe of example 2 was used to treat surfaces (stainless steel discs) which had been heavily soiled with *E. coli* (ATCC 10536). The treatment was 30 seconds. Both a wipe without lactam and a wipe with lactam were tested, along with a water blank disc control. The level of bacterial coverage was counted by measuring the resultant level of bacteria remaining on the stainless steel discs.

The results show that the lactam wipe reduced the level of *E. coli* compared to the same wipe without lactam. The results were statistically significant.

| | **Water blank control treatment** | **Control Wipe (without lactam)** | **Invention Wipe (with lactam)** |
|---|---|---|---|
| Mean Log10 bacterial count | 6.13 | 2.64 | 1.84 |
| Standard Deviation | 0.39 | 0.06 | 0.27 |

### Results of lactam wipes on S. aureus

The wipe of example 2 was used to treat surfaces (stainless steel discs) which had been heavily soiled with *S*. *aureus.* The treatment was 3 x 10 seconds. Both a wipe without lactam and a wipe with lactam were tested, along with a water blank disc control. The level of bacterial coverage was counted by measuring the resultant level of bacteria remaining on the stainless steel discs.

The results show that the lactam wipe reduced the level of *S*. *aureus* compared to the same wipe without lactam. The results were statistically significant.

| | **Water blank control treatment** | **Control Wipe (without lactam)** | **Invention Wipe (with lactam)** |
|---|---|---|---|
| Mean Log10 bacterial count | 5.44 | 4.40 | 3.13 |
| Standard Deviation | 0.08 | 0.4 | 0.42 |

### Example 4 - Example of self-preservation

The wipe of example 2 was tested to see if the wipe itself could be considered as self-preserving. Two types of wipe having the formulation of example 2 were produced on 2 different carriers (viscose and polyethylene terephthalate) and left for 48 hours after being challenged by *S*. *aureus.* The level of bacteria on the wipe after 48hrs was measured using fluorescence.

| | **Viscose Control** | **Viscose Control + lactam** | **PET Control** | **PET Control + lactam** |
|---|---|---|---|---|
| Fluorescence | 955.7 | 53.5 | 993.9 | 94.7 |

| | | | | |
|---|---|---|---|---|
| Standard deviation | 30.8 | 1.2 | 153.0 | 34.0 |

## Claims

1. A wipe suitable for application to a hard surface comprising from 0.0001 to 5 wt.% of a lactam, wherein the lactam is a lactam selected from: 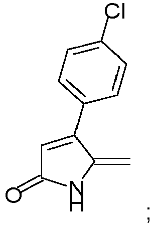 4-(4-chlorophenyl)-5-methylene-pyrrol-2-one; and 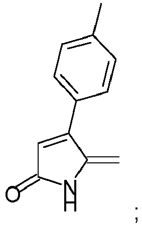 5-methylene-4-(p-tolyl)pyrrol-2-one.

2. A wipe according to claim 1 comprising:-
a) from 0.001 to 5 wt.% of a lactam; and,
b) from 0.1 to 20 wt.% of a surfactant.

3. A wipe according to claim 1 or claim 2 comprising:-
a) from 0.001 to 5 wt.% of a lactam;
b) from 0.1 to 20 wt.%, preferably from 0.5 to 10 wt.%, more preferably from 1 to 8 wt.% of a surfactant; and,
c) from 0.01 to 2.5 wt.% of a chelating agent.

4. A wipe according to any preceding claim, wherein the lactam is present at a level of from 0.0001 to 2.5 wt.%, preferably from 0.0001 to 1 wt.%, more preferably from 0.001 to 1 wt.%.

5. A wipe according to any preceding claim, wherein the lactam is a lactam selected from: 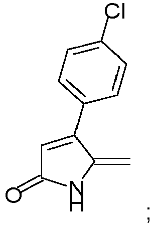 4-(4-chlorophenyl)-5-methylene-pyrrol-2-one.

6. A wipe according to any preceding claim, wherein the lactam is in encapsulated form.

7. A wipe according to any preceding claim, wherein the surfactant is selected from anionic and nonionic surfactants, preferably nonionic surfactants, more preferably alcohol ethoxylates.

8. A wipe according to any preceding claim, wherein the chelating agent is citric acid; EDTA (ethylenediaminetetraacetic acid) or a phosphonic acid.

9. A wipe according to any preceding claim, additionally comprising any one or more of the following: buffers, antifoam agents, perfumes, and, preservative.

10. Use of a lactam in a wipe product to enhance preservation of said wipe product, wherein the lactam is selected from: 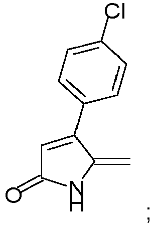 4-(4-chlorophenyl)-5-methylene-pyrrol-2-one; and 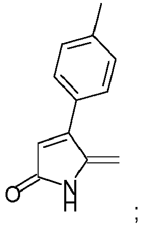 5-methylene-4-(p-tolyl)pyrrol-2-one.

11. Use of a wipe according to any one of claims 1 to 9 on a surface to reduce microorganism buildup on said surface.

12. Use according to claims 10 or 11, wherein the lactam has the following structure: 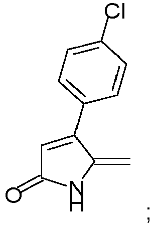 4-(4-chlorophenyl)-5-methylene-pyrrol-2-one.

## Patentansprüche

1. Wischtuch, geeignet zur Anwendung auf einer harten Oberfläche, umfassend 0,0001 bis 5 Gew.-% eines Lactams, wobei das Lactam ein Lactam darstellt, das unter 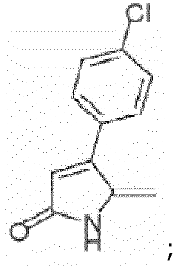 4-(4-Chlorphenyl)-5-methylen-pyrrol-2-on; und 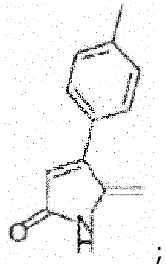 5-Methylen-4-(p-tolyl)pyrrol-2-on ausgewählt ist.

2. Wischtuch nach Anspruch 1, umfassend:
a) 0,001 bis 5 Gew.-% eines Lactams; und
b) 0,1 bis 20 Gew.-% eines Tensids.

3. Wischtuch nach Anspruch 1 oder Anspruch 2, umfassend:
a) 0,001 bis 5 Gew.-% eines Lactams;
b) 0,1 bis 20 Gew.-%, bevorzugt 0,5 bis 10 Gew.-%, bevorzugter 1 bis 8 Gew.-% eines Tensids; und
c) 0,01 bis 2,5 Gew.-% eines Komplexbildners.

4. Wischtuch nach einem vorhergehenden Anspruch, wobei das Lactam in einer Menge von 0,0001 bis 2,5 Gew.-%, bevorzugt von 0,0001 bis 1 Gew.-%, bevorzugter von 0,001 bis 1 Gew.-%, vorliegt.

5. Wischtuch nach einem vorhergehenden Anspruch, wobei das Lactam ein Lactam darstellt, das unter 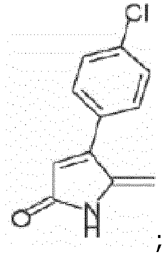 4-(4-Chlorphenyl)-5-methylen-pyrrol-2-on ausgewählt ist.

6. Wischtuch nach einem vorhergehenden Anspruch, wobei das Lactam in eingekapselter Form vorliegt.

7. Wischtuch nach einem vorhergehenden Anspruch, wobei das Tensid unter anionischen und nichtionischen Tensiden, bevorzugt nichtionischen Tensiden, bevorzugter Alkoholethoxylaten, ausgewählt ist.

8. Wischtuch nach einem vorhergehenden Anspruch, wobei der Komplexbildner Citronensäure, EDTA (Ethylendiamintetraessigsäure) oder eine Phosphonsäure ist.

9. Wischtuch nach einem vorhergehenden Anspruch, das zusätzlich eine oder mehrere der folgenden Substanzen enthält: Puffer, Antischaummittel, Parfüme und Konservierungsmittel.

10. Verwendung eines Lactams in einem Wischtuchprodukt zur Verbesserung der Erhaltung des Wischtuchprodukts, wobei das Lactam unter 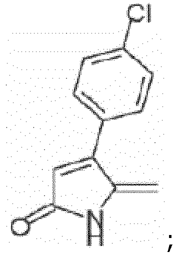 4-(4-Chlorphenyl)-5-methylen-pyrrol-2-on; und 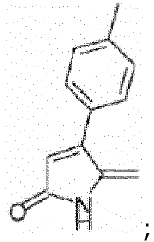 5-Methylen-4-(p-tolyl)pyrrol-2-on ausgewählt ist.

11. Verwendung eines Wischtuchs nach einem der Ansprüche 1 bis 9 auf einer Oberfläche zur Verminderung der Ansammlung von Mikroorganismen auf der Oberfläche.

12. Verwendung nach den Ansprüchen 10 oder 11, wobei das Lactam die folgende Struktur aufweist: 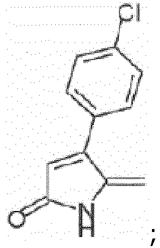 4-(4-Chlorphenyl)-5-methylen-pyrrol-2-on.

## Revendications

1. Lingette applicable à une surface dure, comprenant de 0,0001 à 5 % en poids d'un lactame, dans laquelle le lactame est un lactame choisi parmi : 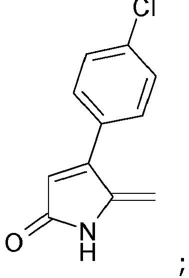 4-(4-chlorophényl)-5-méthylène-pyrrol-2-one ; et 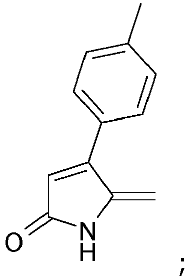 5-méthylène-4-(p-tolyl)pyrrol-2-one.

2. Lingette selon la revendication 1, comprenant :
a) de 0,001 à 5 % en poids d'un lactame ; et
b) de 0,1 à 20 % en poids d'un tensioactif.

3. Lingette selon la revendication 1 ou la revendication 2, comprenant :
a) de 0,001 à 5 % en poids d'un lactame ;
b) de 0,1 à 20 % en poids, de préférence de 0,5 à 10 % en poids, mieux encore de 1 à 8 % en poids d'un tensioactif ; et
c) de 0,01 à 2,5 % en poids d'un agent chélatant.

4. Lingette selon l'une quelconque des revendications précédentes, dans laquelle le lactame est présent à raison de 0,0001 à 2,5 % en poids, de préférence de 0,0001 à 1 % en poids, mieux encore de 0,001 à 1 % en poids.

5. Lingette selon l'une quelconque des revendications précédentes, dans laquelle le lactame est un lactame choisi parmi : 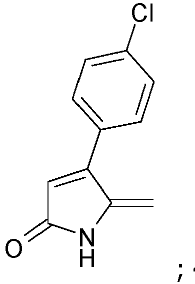 4-(4-chlorophényl)-5-méthylène-pyrrol-2-one.

6. Lingette selon l'une quelconque des revendications précédentes, dans laquelle le lactame est sous une forme encapsulée.

7. Lingette selon l'une quelconque des revendications précédentes, dans laquelle le tensioactif est choisi parmi les tensioactifs anioniques et non-ioniques, de préférence les tensioactifs non-ioniques, mieux encore les alcools éthoxylés.

8. Lingette selon l'une quelconque des revendications précédentes, dans laquelle l'agent chélatant est l'acide citrique ; l'EDTA (acide éthylènediaminetétraacétique) ou un acide phosphonique.

9. Lingette selon l'une quelconque des revendications précédentes, comprenant de plus l'un quelconque ou plusieurs des suivants : tampons, agents antimoussants, parfums, et conservateur.

10. Utilisation d'un lactame dans un produit en lingette pour amplifier la conservation dudit produit en lingette, dans laquelle le lactame est choisi parmi : 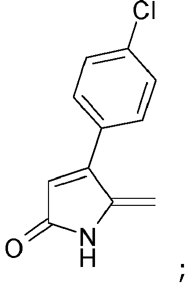 4-(4-chlorophényl)-5-méthylène-pyrrol-2-one ; et 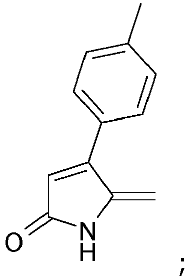 5-méthylène-4-(p-tolyl)pyrrol-2-one.

11. Utilisation d'une lingette selon l'une quelconque des revendications 1 à 9 sur une surface pour réduire l'accumulation de microorganismes sur ladite surface.

12. Utilisation selon la revendication 10 ou 11, dans laquelle le lactame a la structure suivante : 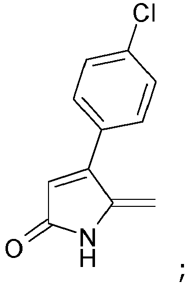 4-(4-chlorophényl)-5-méthylène-pyrrol-2-one.
